(19) Europäisches Patentamt
European Patent Office
Office européen des brevets

(11) Veröffentlichungsnummer : **0 054 810**
**B1**

(12) # EUROPÄISCHE PATENTSCHRIFT

(45) Veröffentlichungstag der Patentschrift : **30.07.86**

(21) Anmeldenummer : **81110184.9**

(22) Anmeldetag : **05.12.81**

(51) Int. Cl.[4] : **C 12 P 33/02**// C07J1/00, C07J5/00, C07J7/00, C07J63/00, C07J71/00

(54) **Verfahren zur Herstellung von 3-Oxo-delta-1,4-steroiden.**

(30) Priorität : **23.12.80 DE 3049399**

(43) Veröffentlichungstag der Anmeldung : **30.06.82 Patentblatt 82/26**

(45) Bekanntmachung des Hinweises auf die Patenterteilung : **30.07.86 Patentblatt 86/31**

(84) Benannte Vertragsstaaten : **AT BE CH DE FR GB IT LI LU NL SE**

(56) Entgegenhaltungen :
**DE-B- 1 070 176**
**DE-B- 1 093 792**
**GB-A- 908 976**
**CHEMICAL ABSTRACTS, Band 73, Nr. 15, 12. Oktober 1970, Seite 104, Nr. 74110q, Columbus, Ohio, USA M. NAGASAWA et al.: "Microbial transformation of sterols. V. Inhibitors of microbial degradation of cholesterol"**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **Weber, Alfred, Dr.**
**Schützallee 56**
**D-1000 Berlin 37 (DE)**
Erfinder : **Kennecke, Mario, Dr.**
**Taubertstrasse 31 f**
**D-1000 Berlin 33 (DE)**

**EP 0 054 810 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft das in den Patentansprüchen gekennzeichnete Verfahren.

Die Fermentation von 3-Oxo-$\Delta^4$-steroiden mit lebenden Kulturen bekannter Steroid-$\Delta^1$-dehydrierern wie Bacillus lentus ATCC 13 805, Bacillus sphaericus ATCC 7 054, ATCC 7 055 und ATCC 12 488, Bacillus subtilis NRRL B 558, Arthrobacter simplex sowie Norcardia corallina ATCC 4 273 und 4 275 verläuft oft unbefriedigend, teils weil die Umsetzungsgeschwindigkeit zu gering ist oder keine ausreichende Umsetzung des Substrates beobachtet wird, teils weil das Substrat stark metabolisiert wird.

Es wurde nun gefunden, daß man die mikrobiologische $\Delta^1$-Dehydrierung von 3-Oxo-$\Delta^4$-steroiden sehr gut durchführbar ist, wenn man zur Dehydrierung dieses Substrates einen Mikroorganismus der Spezies Arthrobacter simplex ATCC 6 946 verwendet und die Umsetzung in Gegenwart von 0,04 g bis 0,12 g Kobalt (II)-ionen pro Liter Kultur durchführt.

Es ist seit langem bekannt, daß Arthrobacter simplex ATCC 6 946 — der früher als Corynebacterium simplex bezeichnet wurde — die Fähigkeit zu $\Delta^1$-Dehydrierung von Steroiden besitzt. Er wird in der Technik oft verwendet, da er gegenüber den meisten übrigen der obengenannten Steroid-$\Delta^1$-dehydrierern den Vorzug hat, daß die Umsetzung mit diesen Stamm wesentlich rascher abläuft, als mit jenen Mikoorganismen ; andererseits hat Arthrobacter simplex aber auch zwei Nachteile :

1. Er neigt oft dazu, bei längerer Fermentationszeit Steroide zu metabolisieren, was zu nicht unerheblichen Ausbeuteverlusten an gewünschtem Verfahrensprodukt führen kann und

2. Er neigt oft dazu, die Reaktion zu stoppen, wenn noch 1 bis 3 % Ausgangssteroid in der Kulturbrühe vorhanden ist. Dies erschwert die Herstellung der Verfahrensprodukte in einer Reinheit, wie sie für Arzneimittelwirkstoffe unerläßlich ist, oft ganz erheblich.

Diese Nachteile kann man vermeiden, wenn man die $\Delta^1$-Dehydrierung mit Arthrobacter simplex ATCC 6 946 in Gegenwart von 0,04 g bis 0,12 g kobalt (II)-ionen pro Liter Kulturbrühe durchführt. Eine geringere Konzentration an Kobaltionen bringt keinen nennenswerten Effekt, eine höhere Konzentration an Kobalt (II)-ionen wirkt inhibierend. Geeignete Kobalt-(II)-ionen liefernde Agenzien sind wasserlösliche Kobaltsalze wie zum Beispiel $CoCl_2$, $CoNO_3$, $CoSO_4$ oder $CoSO_4$, $7H_2O$. Die Anionen dieser Salze sind für die Durchführbarkeit des Verfahrens ohne Bedeutung, da die Kobalt (II)-salze ja in dem Kulturmedium dissoziieren.

Im übrigen wird das erfindungsgemäße Verfahren unter den Bedingungen durchgeführt, die man üblicherweise zur $\Delta^1$-Dehydrierung von Steroiden mit Mikroorganismen der Spezies Arthrobacter simplex anwendet.

Unter den für diesen Mikroorganismus üblicherweise verwendeten Kulturbedingungen wird in einem geeigneten Nährmedium unter Belüften eine Submerskultur angezüchtet. Dann setzt man den Kulturen das Substrat (in einem geeigenten Lösungsmittel gelöst oder vorzugsweise in emulgierter Form) zu und fermentiert, bis eine maximale Substratumwandlung erreicht ist.

Geeignete Substratlösungsmittel sind beispielsweise Methanol, Äthanol, Glykolmonomethyläther, Dimethylformamid, Dimethylsulfoxyd, oder Hexamethylphosphorsäretriamid. Die Emulgierung des Substrats kann beispielsweise bewirkt werden, in dem man dieses in mikronisierter Form oder in einem mit Wasser mischbaren Lösungsmittel (wie Methanol, Äthanol, Aceton, Glykolmonomethyläther, Dimethylformamid oder Dimethylsulfoxyd) gelöst unter starker Turbulenz in (vorzugsweise entkalktem) Wasser, welches die üblichen Emulgationshilfen enthält, eindüst. Geeignete Emulgationshilfen sind nichtionogene Emulgatoren, wie zum Beispiel Äthylenoxydaddukte oder Fettsäureester von Polyglykolen. Als geeignete Emulgatoren seien die handelsüblichen Netzmittel Tegin (R), Tagat (R), Tween (R) und Span (R) beispielsmäßig genannt.

Die optimale Substratkonzentration, Substratzugabezeit und Fermentationsdauer ist von der Art des verwendeten Mikroorganismus abhängig. Diese Größen müssen, wie dies bei mikrobiologischen Steroidumwandlungen allgemein erforderlich ist, im Einzelfall durch Vorversuche, wie sie dem Fachmann geläufig sind, ermitteltwerden.

Es ist für den Fachmann überraschend, daß bei der Fermentation unter Zusatz von 0,04 g bis 0,12 g Kobalt (II)-ionen der Reaktionsablauf beschleunigt wird und vollständiger in der gewünschten Richtung abläuft, als ohne Verwendung dieses Zusatzes. Zwar ist bekannt, daß man bei der Fermentation Kobalt (II)-ionen als Spurenelemente zusetzen kann, ohne den Reaktionsablauf wesentlich zu beeinträchtigen (DAS 10 70 176). Verwendet man aber größere Mengen Kobalt (II)-ionen, so wirken diese nach dem bekannten Stande der Technik als Inhibitoren. Hiervon macht der Fachmann beispielsweise Gebrauch, wenn er die Seitenkette von Sterinen abbauen und gleichzeitig die 9α-Hydroxylierung des Steroidkerns unterdrücken will (Agr. Biol. Chem. Band 34, Nr 6, 1970, 838 ff).

Die nachfolgenden Ausführungsbeispiele dienen zur Erläuterung des erfindungsgemäßen Verfahrens.

Das in dem nachfolgenden Ausführungsbeispiel verwendete Proteinhydrolysat wurde aus Federmehl gemäß der Vorschrift von Organic Synthesis, Col. Vol. I, Seite 194 hergestellt, jedoch wurde nach

erfolgter Hydrolyse mittels Ammoniak neutralisiert und sterilfiltriert. Das erhaltene Filtrat wurde ohne weitere Reinigung als « Proteinhydrolysat » verwendet.

## Beispiel 1

a) Ein 2 l-Erlenmeyerkolben mit 1 l steriler Nährlösung enthaltend

0,5 % Cornsteep liquor
0,05 % Glucosemonohydrat
0,1 % Hefeextrakt
— eingestellt auf pH 7,0 —

wird mit einer Abschwemmung einer Trockenkultur von Arthrobacter simplex ATCC 6946 beimpft und bei 30 °C 72 Stunden mit 180 Umdrehungen pro Minute geschüttelt.

b) Ein 50 l Fermenter mit 30 l steriler Nährlösung enthaltend

0,5 % Cornsteep liquor
0,05 % Glucosemonohydrat
0,1 % Hefeextrakt
4 ml Silikon SH
4 ml Pluronic
— eingestellt auf pH 7,0 —

wird mit 1 l Arthrobacter simplex-Anzuchtskultur beimpft und diese Vorkultur bei 30 °C unter Belüftung (2 $m^3$ pro Stunde) und Rühren (220 Umdrehungen pro Minute) 24 Stunden lang incubiert.

c) 7,5 g 9β,11β-Epoxi-17α-hydroxy-16β-methyl-4-pregnen-3,20-dion werden in 300 ml Äthylenglykolmonomethyläther gelöst und anschließend sterilfiltriert.

d) 12 g $CoSO_4 \cdot 7H_2O$ und 12 g $(NH_4)_2SO_4$ werden in 500 ml Wasser gelöst, auf einen pH-Wert von 6,5 eingestellt und sterilisiert.

e) Ein 50 l-Fermenter wird mit 30 l steriler Nährlösung enthaltend

0,5 % Cornsteep liquor
0,05 % Glucosemonohydrat
0,3 % « Proteinhydrolysat »
4 ml Silikon SH
4 ml Pluronic
— eingestellt auf pH 7,0 —

beschickt, mit 3 l Arthrobacter simplex Vorkultur beimpft und unter Belüften (2 $m^3$ pro Stunde) und Rühren (220 Umdrehungen pro Minute) 6 Stunden lang bei 30 °C incubiert. Dann setzt man der Kultur die in Abschnitt c) hergestellte sterile 9β,11β-Epoxi-17α-hydroxy-16β-methyl-4-pregnen-3,20-dion-Lösung und die in Abschnitt d) hergestellten Zusätze zu. Danach wird weiter 20 Stunden lang fermentiert.

Nach erfolgter Fermentation wird die mit 5 l Äthylenchlorid stabilisierte Kulturbrühe 3 mal mit je 20 l Äthylenchlorid extrahiert und der Äthylenchlorid-Extrakt im Rotationsverdampfer bei max. 50 °C unter Vakuum eingeengt. Anschließend erfolgt eine Reinigung durch Chromatographie an Aluminiumoxyd.

Man erhält 5,8 g 9β,11β-Epoxi-17α-hydroxy-16β-methyl-1,4-pregnadien-3,20-dion vom Schmelzpunkt 212/213-214 °C.

## Beispiel 2

a) Ein 2 l-Erlenmeyerkolben mit 1 l steriler Nährlösung enthaltend

0,5 % Cornsteep liquor
0,05 % Glucosemonohydrat
0,1 % Hefeextrakt
— eingestellt auf pH 7,0 —

wird mit einer Abschwemmung einer Trockenkultur von Arthrobacter simplex ATCC 6946 beimpft und bei 30 °C 72 Stunden mit 180 Umdrehungen pro Minute geschüttelt.

b) Ein 50 l Fermenter mit 30 l steriler Nährlösung enthaltend

0,5 % Cornsteep liquor
0,05 % Glucosemonohydrat
0,1 % Hefeextrakt
4 ml Silikon SH
4 ml Pluronic
— eingestellt auf pH 7,0 —

wird mit 1 l Arthrobacter simplex-Anzuchtskultur beimpft und diese Vorkultur bei 30 °C unter Belüftung (2 $m^3$ pro Stunde) und Rühren (220 Umdrehungen pro Minute) 24 Stunden lang incubiert.

c) 7,5 g 17α-Hydroxy-16β-methyl-4,9(11)-pregnadien-3,20-dion werden in 150 ml Äthylenglykolmonomethyläther bei 60 °C gelöst und anschließend sterilfiltriert.

d) 18 g $CoSO_4 \cdot 7H_2O$ und 18 g $(NH_4)_2SO_4$ werden in 500 ml Wasser gelöst, auf einen pH-Wert von 6,5 eingestellt und sterilisiert.

e) Ein 50 l-Fermenter wird mit 30 l steriler Nährlösung enthaltend

0,5 % Cornsteep liquor
0,05 % Glucosemonohydrat
0,3 % « Proteinhydrolysat »
4 ml Silikon SH
4 ml Pluronic
— eingestellt auf pH 7,0 —

beschickt, mit 3 l Arthrobacter simplex Vorkultur beimpft und unter Belüften (2 $m^3$ pro Stunde) und Rühren (220 Umdrehungen pro Minute) 6 Stunden lang bei 30 °C incubiert. Dann setzt man der Kultur die in Abschnitt c) hergestellte sterile 17α-Hydroxy-16β-methyl-4,9(11)-pregnadien-3,20-dion-Lösung und die in Abschnitt d) hergestellten Zusätze zu. Danach wird weiter 24 Stunden lang fermentiert.

Nach erfolgter Fermentation wird die mit 5 l Äthylenchlorid stabilisierte Kulturbrühe 3 mal mit je 20 l Äthylenchlorid extrahiert und der Äthylenchlorid-Extrakt im Rotationsverdampfer bei max. 50 °C unter Vakuum eingeengt. Anschließend erfolgt eine Reinigung durch Chromatographie an Aluminiumoxyd.

Man erhält 6,2 g 17α-Hydroxy-16β-methyl-1,4,9(11)-pregnatrien-3,20-dion vom Schmelzpunkt 177-178 °C.

## Beispiel 3

a) Ein 21-Erlenmeyerkolben mit 1 l steriler Nährlösung enthaltend

0,5 % Cornsteep liquor
0,05 % Glucosemonohydrat
0,1 % Hefeextrakt
— eingestellt auf pH 7,0 —

wird mit einer Abschwemmung einer Trockenkultur von Arthrobacter simplex ATCC 6946 beimpft und bei 30 °C 72 Stunden mit 180 Umdrehungen pro Minute geschüttelt.

b) Ein 30 l Fermenter mit 20 l steriler Nährlösung enthaltend

0,5 % Cornsteep liquor
0,05 % Glucosemonohydrat
0,1 % Hefeextrakt
4 ml Silikon SH
4 ml Pluronic
— eingestellt auf pH 7,0 —

wird mit 1 l Arthrobacter simplex-Anzuchtskultur beimpft und diese Vorkultur bei 30 °C unter Belüftung (2 $m^3$ pro Stunde) und Rühren (220 Umdrehungen pro Minute) 24 Stunden lang incubiert.

c) 6 g 17α,21-Dihydroxy-16-methylen-4-pregnen-3,11,20-trion werden in 300 ml Hexamethylphosphorsäuretriamid gelöst und anschließend sterilfiltriert.

d) 9 g $CoSO_4 \cdot 7H_2O$ und 9 g $(NH_4)_2SO_4$ werden in 500 ml Wasser gelöst, auf einen pH-Wert von 6,5 eingestellt und sterilisiert.

e) Ein 30 l-Fermenter wird mit 20 l steriler Nährlösung enthaltend

0,5 % Cornsteep liquor
0,05 % Glucosemonohydrat
0,3 % « Proteinhydrolysat »
4 ml Silikon SH
4 ml Pluronic
— eingestellt auf pH 7,0 —

beschickt, mit 1,5 l Arthrobacter simplex Vorkultur beimpft und unter Belüften (2 $m^3$ pro Stunde) und Rühren (220 Umdrehungen pro Minute) 6 Stunden lang bei 30 °C incubiert. Dann setzt man der Kultur die in Abschnitt c) hergestellte sterile 21-Acetoxy-17α-hydroxy-16-methylen-4-pregnen-3,11,20-trion-Lösung und die in Abschnitt d) hergestellten Zusätze zu. Danach wird weiter 16 Stunden lang fermentiert.

Nach erfolgter Fermentation wird die mit 3 l Äthylenchlorid stabilisierte Kulturbrühe 3 mal mit je 10 l Äthylenchlorid extrahiert und der Äthylenchlorid-Extrakt im Rotationsverdampfer bei max. 50 °C unter Vakuum eingeengt. Anschließend erfolgt eine Reinigung durch Chromatographie an Aluminiumoxyd.

Man erhält 4,5 g 17α,21-Dihydroxy-16-methylen-1,4-pregnadien-3,11,20-trion vom Schmelzpunkt 218-219 °C.

## Beispiel 4

a) Ein 21-Erlenmeyerkolben mit 1 l steriler Nährlösung enthaltend

0,5 % Cornsteep liquor
0,05 % Glucosemonohydrat
0,1 % Hefeextrakt
— eingestellt auf pH 7,0 —

wird mit einer Abschwemmung einer Trockenkultur von Arthrobacter simplex ATCC 6946 beimpft und bei 30 °C 72 Stunden mit 180 Umdrehungen pro Minute geschüttelt.

b) Ein 30 l Fermenter mit 20 l steriler Nährlösung enthaltend

0,5 % Cornsteep liquor
0,05 % Glucosemonohydrat
0,1 % Hefeextrakt
4 ml Silikon SH
4 ml Pluronic
— eingestellt auf pH 7,0 —

wird mit 1 l Arthrobacter simplex-Anzuchtskultur beimpft und diese Vorkultur bei 30 °C unter Belüftung (2 $m^3$ pro Stunde) und Rühren (220 Umdrehungen pro Minute) 24 Stunden lang incubiert.

c) 6,0 g 11β, 17aα, 21-Trihydroxy-D-homo-4-pregnen-3,20-dion werden in 150 ml Hexamethylphosphorsäuretriamid gelöst und anschließend sterilfiltriert.

d) 9 g $CoSO_4 \cdot 7H_2O$ und 9 g $(NH_4)_2SO_4$ werden in 400 ml Wasser gelöst, auf einen pH-Wert von 6,5 eingestellt und sterilisiert.

e) Ein 30 l-Fermenter wird mit 20 l steriler Nährlösung enthaltend

0,5 % Cornsteep liquor
0,05 % Glucosemonohydrat
0,3 % « Proteinhydrolysat »
4 ml Silikon SH
4 ml Pluronic
— eingestellt auf pH 7,0 —

beschickt, mit 1,5 l Arthrobacter simplex Vorkultur beimpft und unter Belüften (2 $m^3$ pro Stunde) und Rühren (220 Umdrehungen pro Minute) 6 Stunden lang bei 30 °C incubiert. Dann setzt man der Kultur die in Abschnitt c) hergestellte sterile 11β, 17aα, 21-Trihydroxy-D-homo-4-pregnen-3,20-dion-Lösung und die in Abschnitt d) hergestellten Zusätze zu. Danach wird weiter 18 Stunden lang fermentiert.

Nach erfolgter Fermentation wird die mit 3 l Äthylenchlorid stabilisierte Kulturbrühe 3 mal mit je 15 l Äthylenchlorid extrahiert und der Äthylenchlorid-Extrakt im Rotationsverdampfer bei max. 50 °C unter Vakuum eingeengt. Anschließend erfolgt eine Reinigung durch Chromatographie an Aluminiumoxyd.

Man erhält 5,0 g 11β, 17aα, 21-Trihydroxy-D-homo-1,4-pregnadien-3,20-dion vom Schmelzpunkt 248-249 °C (Zersetzung).

Beispiel 5

a) Ein 2 l-Erlenmeyerkolben mit 1 l steriler Nährlösung enthaltend

0,5 % Cornsteep liquor
0,05 % Glucosemonohydrat
0,1 % Hefeextrakt
— eingestellt auf pH 7,0 —

wird mit einer Abschwemmung einer Trockenkultur von Arthrobacter simplex ATCC 6946 beimpft und bei 30 °C 72 Stunden mit 180 Umdrehungen pro Minute geschüttelt.

b) Ein 50 l Fermenter mit 30 l steriler Nährlösung enthaltend

0,5 % Cornsteep liquor
0,05 % Glucosemonohydrat
0,1 % Hefeextrakt
4 ml Silikon SH
4 ml Pluronic
— eingestellt auf pH 7,0 —

wird mit 1 l Arthrobacter simplex-Anzuchtskultur beimpft und diese Vorkultur bei 30 °C unter Belüftung (2 $m^3$ pro Stunde) und Rühren (220 Umdrehungen pro Minute) 24 Stunden lang incubiert.

c) 15 g 6α-Fluor-11β, 21-dihydroxy-16α-methyl-4-pregnen-3,20-dion werden in 300 ml Hexamethylphosphorsäuretriamid gelöst und anschließend sterilfiltriert.

d) 12 g $CoSO_4 \cdot 7H_2O$ und 12 g $(NH_4)_2SO_4$ werden in 500 ml Wasser gelöst, auf einen pH-Wert von 6,5 eingestellt und sterilisiert.

e) Ein 50 l-Fermenter wird mit 30 l steriler Nährlösung enthaltend

0,5 % Cornsteep liquor
0,05 % Glucosemonohydrat
0,3 % « Proteinhydrolysat »
4 ml Silikon SH
4 ml Pluronic
— eingestellt auf pH 7,0 —

beschickt, mit 3 l Arthrobacter simplex Vorkultur beimpft und unter Belüften (2 $m^3$ pro Stunde) und Rühren (220 Umdrehungen pro Minute) 6 Stunden lang bei 30 °C incubiert. Dann setzt man der Kultur die in Abschnitt c) hergestellte sterile 6α-Fluor-11β, 21-dihydroxy-16α-methyl-4-pregnen-3,20-dion-Lösung und die in Abschnitt d) hergestellten Zusätze zu. Danach wird weiter 22 Stunden lang fermentiert.

Nach erfolgter Fermentation wird die mit 5 l Äthylenchlorid stabilisierte Kulturbrühe 3 mal mit je 20 l Äthylenchlorid extrahiert und der Äthylenchlorid-Extrakt im Rotationsverdampfer bei max. 50 °C unter Vakuum eingeengt. Anschließend erfolgt eine Reinigung durch Chromatographie an Aluminiumoxyd.

Man erhält 13,1 g 6α-Fluor-11β, 21-dihydroxy-16α-methyl-1,4-pregnadien-3,20-dion vom Schmelzpunkt 180/181°-182 °C.

Beispiel 6

a) Ein 2 l-Erlenmeyerkolben mit 1 l steriler Nährlösung enthaltend

0,5 % Cornsteep liquor
0,05 % Glucosemonohydrat
0,1 % Hefeextrakt
— eingestellt auf pH 7,0 —

wird mit einer Abschwemmung einer Trockenkultur von Arthrobacter simplex ATCC 6946 beimpft und bei 30 °C 72 Stunden mit 180 Umdrehungen pro Minute geschüttelt.

b) Ein 50 l Fermenter mit 30 l steriler Nährlösung enthaltend

0,5 % Cornsteep liquor
0,05 % Glucosemonohydrat
0,1 % Hefeextrakt
4 ml Silikon SH
4 ml Pluronic
— eingestellt auf pH 7,0 —

wird mit 1 l Arthrobacter simplex-Anzuchtskultur beimpft und diese Vorkultur bei 30 °C unter Belüftung (2 $m^3$ pro Stunde) und Rühren (220 Umdrehungen pro Minute) 24 Stunden lang incubiert.

c) 15 g 11β, 21-Dihydroxy-16α, 17α-isopropylidendioxy-4-pregnen-3,20-dion werden in 300 ml Äthylenglykolmonomethyläther gelöst und anschließend sterilfiltriert.

d) 12 g $CoSO_4 \cdot 7H_2O$ und 12 g $(NH_4)_2SO_4$ werden in 500 ml Wasser gelöst, auf einen pH-Wert von 6,5 eingestellt und sterilisiert.

e) Ein 50 l-Fermenter wird mit 30 l steriler Nährlösung enthaltend

0,5 % Cornsteep liquor
0,05 % Glucosemonohydrat
0,3 % « Proteinhydrolysat »
4 ml Silikon SH
4 ml Pluronic
— eingestellt auf pH 7,0 —

beschickt, mit 3 l Arthrobacter simplex Vorkultur beimpft und unter Belüften (2 $m^3$ pro Stunde) und Rühren (220 Umdrehungen pro Minute) 6 Stunden lang bei 30 °C incubiert. Dann setzt man der Kultur die in Abschnitt c) hergestellte sterile 11β, 21-Dihydroxy-16α, 17α-isopropylidendioxy-4-pregnen-3,20-dion-Lösung und die in Abschnitt d) hergestellten Zusätze zu. Danach wird weiter 20 Stunden lang fermentiert.

Nach erfolgter Fermentation wird die mit 5 l Äthylenchlorid stabilisierte Kulturbrühe 3 mal mit je 20 l Äthylenchlorid extrahiert und der Äthylenchlorid-Extrakt im Rotationsverdampfer bei max. 50 °C unter Vakuum eingeengt. Anschließend erfolgt eine Reinigung durch Chromatographie an Aluminiumoxyd.

Man erhält 13,6 g 11β, 21-Dihydroxy-16α, 17α-isopropylidendioxy-1,4-pregnadien-3,20-dion vom Schmelzpunkt 241-242,5 °C.

## Beispiel 7

a) Ein 2 l-Erlenmeyerkolben mit 1 l steriler Nährlösung enthaltend

0,5 % Cornsteep liquor
0,05 % Glucosemonohydrat
0,1 % Hefeextrakt
— eingestellt auf pH 7,0 —

wird mit einer Abschwemmung einer Trockenkultur von Arthrobacter simplex ATCC 6946 beimpft und bei 30 °C 72 Stunden mit 180 Umdrehungen pro Minute geschüttelt.

b) Ein 50 l Fermenter mit 30 l steriler Nährlösung enthaltend

0,5 % Cornsteep liquor
0,05 % Glucosemonohydrat
0,1 % Hefeextrakt
4 ml Silikon SH
4 ml Pluronic
— eingestellt auf pH 7,0 —

wird mit 1 l Arthrobacter simplex-Anzuchtskultur beimpft und diese Vorkultur bei 30 °C unter Belüftung (2 $m^3$ pro Stunde) und Rühren (220 Umdrehungen pro Minute) 24 Stunden lang incubiert.

c) 21 g 11β, 17α, 21-Trihydroxy-6α-methyl-4-pregnen-3,20-dion werden in 300 ml Äthylenglykolmonomethyläther gelöst und anschließend sterilfiltriert.

d) 12 g $CoSO_4 \cdot 7H_2O$ und 12 g $(NH_4)_2SO_4$ werden in 500 ml Wasser gelöst, auf einen pH-Wert von 6,5 eingestellt und sterilisiert.

e) Ein 50 l-Fermenter wird mit 30 l steriler Nährlösung enthaltend

0,5 % Cornsteep liquor
0,05 % Glucosemonohydrat
0,3 % « Proteinhydrolysat »
4 ml Silikon SH
4 ml Pluronic
— eingestellt auf pH 7,0 —

beschickt, mit 3 l Arthrobacter simplex Vorkultur beimpft und unter Belüften (2 $m^3$ pro Stunde) und Rühren (220 Umdrehungen pro Minute) 9 Stunden lang bei 30 °C incubiert. Dann setzt man der Kultur die in Abschnitt c) hergestellte sterile 11β,17α,21-Trihydroxy-6α-methyl-4-pregnen-3,20-dion-Lösung und die in Abschnitt d) hergestellten Zusätze zu. Danach wird weiter 7 Stunden lang fermentiert.

Nach erfolgter Fermentation wird die mit 5 l Äthylenchlorid stabilisierte Kulturbrühe 3 mal mit je 20 l Äthylenchlorid extrahiert und der Äthylenchlorid-Extrakt im Rotationsverdampfer bei max. 50 °C unter Vakuum eingeengt. Anschließend erfolgt eine Reinigung durch Chromatographie an Aluminiumoxyd.

Man erhält 19,1 g 11β,17α,21-Trihydroxy-6α-methyl-1,4-pregnadien-3,20-dion vom Schmelzpunkt 235/236-238 °C.

## Beispiel 8

a) Ein 2 l-Erlenmeyerkolben mit 1 l steriler Nährlösung enthaltend

0,5 % Cornsteep liquor
0,05 % Glucosemonohydrat
0,1 % Hefeextrakt
— eingestellt auf pH 7,0 —

wird mit einer Abschwemmung einer Trockenkultur von Arthrobacter simplex ATCC 6946 beimpft und bei 30 °C 72 Stunden mit 180 Umdrehungen pro Minute geschüttelt.

b) Ein 50 l Fermenter mit 30 l steriler Nährlösung enthaltend

0,5 % Cornsteep liquor
0,05 % Glucosemonohydrat
0,1 % Hefeextrakt
4 ml Silikon SH
4 ml Pluronic
— eingestellt auf pH 7,0 —

wird mit 1 l Arthrobacter simplex-Anzuchtskultur beimpft und diese Vorkultur bei 30 °C unter Belüftung (2 $m^3$ pro Stunde) und Rühren (220 Umdrehungen pro Minute) 24 Stunden lang incubiert.

c) 3 g 11β,21-Dihydroxy-17α-propoxymethoxy-4-pregnen-3,20-dion werden in 200 ml Äthylenglycolmonomethyläther gelöst und anschließend sterilfiltriert.

d) 7,5 g $CoSO_4 \cdot 7H_2O$ und 7,5 g $(NH_4)_2SO_4$ werden in 500 ml Wasser gelöst, auf einen pH-Wert von 6,5 eingestellt und sterilisiert.

e) Ein 50 l-Fermenter wird mit 30 l steriler Nährlösung enthaltend

0,5 % Cornsteep liquor
0,05 % Glucosemonohydrat
0,3 % « Proteinhydrolysat »
4 ml Silikon SH
4 ml Pluronic
— eingestellt auf pH 7,0 —

beschickt, mit 3 l Arthrobacter simplex Vorkultur beimpft und unter Belüften (2 $m^3$ pro Stunde) und Rühren (220 Umdrehungen pro Minute) 9 Stunden lang bei 30° C incubiert. Dann setzt man der Kultur die in Abschnitt c) hergestellte sterile 11β,21-Dihydroxy-17α-propoxymethoxy-4-pregnen-3,20-dion-Lösung und die in Abschnitt d) hergestellten Zusätze zu. Danach wird weiter 6 Stunden lang fermentiert.

Nach erfolgter Fermentation wird die mit 5 l Äthylenchlorid stabilisierte Kulturbrühe 3 mal mit je 20 l Äthylenchlorid extrahiert und der Äthylenchlorid-Extrakt im Rotationsverdampfer bei max. 50° C unter Vakuum eingeengt. Anschließend erfolgt eine Reinigung durch Chromatographie an Aluminiumoxyd.

Man erhält 2,4 g 11β,21-Dihydroxy-17α-propoxymethoxy-1,4-pregnadien-3,20-dion vom Schmelzpunkt 121/125-127 °C.

Beispiel 9

a) Ein 2 l-Erlenmeyerkolben mit 1 l steriler Nährlösung enthaltend

0,5 % Cornsteep liquor
0,05 % Glucosemonohydrat
0,1 % Hefeextrakt
— eingestellt auf pH 7,0 —

wird mit einer Abschwemmung einer Trockenkultur von Arthrobacter simplex ATCC 6946 beimpft und bei 30° C 72 Stunden mit 180 Umdrehungen pro Minute geschüttelt.

b) Ein 50 l Fermenter mit 30 l steriler Nährlösung enthaltend

0,5 % Cornsteep liquor
0,05 % Glucosemonohydrat
0,1 % Hefeextrakt
4 ml Silikon SH
4 ml Pluronic
— eingestellt auf pH 7,0 —

wird mit 1 l Arthrobacter simplex-Anzuchtskultur beimpft und diese Vorkultur bei 30 °C unter Belüftung (2 $m^3$ pro Stunde) und Rühren (220 Umdrehungen pro Minute) 24 Stunden lang incubiert.

c) 7,5 g 11β,21-Dihydroxy-17α-isopropoxymethoxy-4-pregnen-3,20-dion werden in 200 ml Äthylenglycolmonomethyläther gelöst und anschließend sterilfiltriert.

d) 7,5 g $CoSO_4 \cdot 7H_2O$ und 7,5 g $(NH_4)_2SO_4$ werden in 500 ml Wasser gelöst, auf einen pH-Wert von 6,5 eingestellt und sterilisiert.

e) Ein 50 l-Fermenter wird mit 30 l steriler Nährlösung enthaltend

0,5 % Cornsteep liquor
0,05 % Glucosemonohydrat
0,3 % « Proteinhydrolysat »
4 ml Silikon SH
4 ml Pluronic
— eingestellt auf pH 7,0 —

beschickt, mit 3 l Arthrobacter simplex Vorkultur beimpft und unter Belüften (2 $m^3$ pro Stunde) und Rühren (220 Umdrehungen pro Minute) 9 Stunden lang bei 30 °C incubiert. Dann setzt man der Kultur die in Abschnitt c) hergestellte sterile 11β,21-Dihydroxy-17α-isopropoxymethoxy-4-pregnen-3,20-dion-Lösung und die in Abschnitt d) hergestellten Zusätze zu. Danach wird weiter 12 Stunden lang fermentiert.

Nach erfolgter Fermentation wird die mit 5 l Äthylenchlorid stabilisierte Kulturbrühe 3 mal mit je 20 l Äthylenchlorid extrahiert und der Äthylenchlorid-Extrakt im Rotationsverdampfer bei max. 50 °C unter Vakuum eingeengt. Anschließend erfolgt eine Reinigung durch Chromatographie an Aluminiumoxyd.

Man erhält 4,2 g 11β,21-Dihydroxy-17α-isopropoxymethoxy-1,4-pregnadien-3,20-dion vom Schmelzpunkt 58/63-65 °C.

Beispiel 10

a) Ein 2 l-Erlenmeyerkolben mit 1 l steriler Nährlösung enthaltend

0,5 % Cornsteep liquor
0,05 % Glucosemonohydrat
0,1 % Hefeextrakt
— eingestellt auf pH 7,0 —

wird mit einer Abschwemmung einer Trockenkultur von Arthrobacter simplex ATCC 6946 beimpft und bei 30 °C 72 Stunden mit 180 Umdrehungen pro Minute geschüttelt.

b) Ein 50 l Fermenter mit 30 l steriler Nährlösung enthaltend

0,5 % Cornsteep liquor
0,05 % Glucosemonohydrat
0,1 % Hefeextrakt
4 ml Silikon SH
4 ml Pluronic
— eingestellt auf pH 7,0 —

wird mit 1 l Arthrobacter simplex-Anzuchtskultur beimpft und diese Vorkultur bei 30 °C unter Belüftung (2 $m^3$ pro Stunde) und Rühren (220 Umdrehungen pro Minute) 24 Stunden lang incubiert.

c) 9 g 11β,21-Dihydroxy-17α-methoxymethoxy-4-pregnen-3,20-dion werden in 300 ml Dimethylformamid gelöst und anschließend sterilfiltriert.

d) 7,5 g $CoSO_4 \cdot 7H_2O$ und 7,5 g $(NH_4)_2SO_4$ werden in 500 ml Wasser gelöst, auf einen pH-Wert von 6,5 eingestellt und sterilisiert.

e) Ein 50 l-Fermenter wird mit 30 l steriler Nährlösung enthaltend

0,5 % Cornsteep liquor
0,05 % Glucosemonohydrat
0,3 % « Proteinhydrolysat »
4 ml Silikon SH
4 ml Pluronic
— eingestellt auf pH 7,0 —

beschickt, mit 3 l Arthrobacter simplex Vorkultur beimpft und unter Belüften (2 $m^3$ pro Stunde) und Rühren (220 Umdrehungen pro Minute) 9 Stunden lang bei 30 °C incubiert. Dann setzt man der Kultur die in Abschnitt c) hergestellte sterile 11β,21-Dihydroxy-17α-methoxymethoxy-4-pregnen-3,20-dion-Lösung und die in Abschnitt d) hergestellten Zusätze zu. Danach wird weiter 6 Stunden lang fermentiert.

Nach erfolgter Fermentation wird die mit 5 l Äthylenchlorid stabilisierte Kulturbrühe 3 mal mit je 20 l Äthylenchlorid extrahiert und der Äthylenchlorid-Extrakt im Rotationsverdampfer bei max. 50 °C unter Vakuum eingeengt. Anschließend erfolgt eine Reinigung durch Chromatographie an Aluminiumoxyd.

Man erhält 5,4 g 11β,21-Dihydroxy-17α-methoxymethoxy-1,4-pregnadien-3,20-dion vom Schmelzpunkt 229/230-231 °C.

Beispiel 11

a) Ein 2 l-Erlenmeyerkolben mit 1 l steriler Nährlösung enthaltend

0,5 % Cornsteep liquor
0,05 % Glucosemonohydrat
0,1 % Hefeextrakt
— eingestellt auf pH 7,0 —

wird mit einer Abschwemmung einer Trockenkultur von Arthrobacter simplex ATCC 6946 beimpft und bei 30 °C 72 Stunden mit 180 Umdrehungen pro Minute geschüttelt.

b) Ein 50 l Fermenter mit 30 l steriler Nährlösung enthaltend

0,5 % Cornsteep liquor
0,05 % Glucosemonohydrat
0,1 % Hefeextrakt
4 ml Silikon SH
4 ml Pluronic
— eingestellt auf pH 7,0 —

wird mit 1 l Arthrobacter simplex-Anzuchtskultur beimpft und diese Vorkultur bei 30 °C unter Belüftung (2 $m^3$ pro Stunde) und Rühren (220 Umdrehungen pro Minute) 24 Stunden lang incubiert.

c) 7,5 g 17β-Hydroxy-17α-methyl-4-androsten-3-on werden in 150 ml Dimethylformamid gelöst und anschließend sterilfiltriert.

d) 12 g $CoSO_4 \cdot 7H_2O$ und 12 g $(NH_4)_2SO_4$ werden in 500 ml Wasser gelöst, auf einen pH-Wert von 6,5 eingestellt und sterilisiert.

e) Ein 50 l-Fermenter wird mit 30 l steriler Nährlösung enthaltend

0,5 % Cornsteep liquor
0,05 % Glucosemonohydrat
0,3 % « Proteinhydrolysat »
4 ml Silikon SH
4 ml Pluronic
— eingestellt auf pH 7,0 —

beschickt, mit 3 l Arthrobacter simplex Vorkultur beimpft und unter Belüften (2 $m^3$ pro Stunde) und Rühren (220 Umdrehungen pro Minute) 6 Stunden lang bei 30 °C incubiert. Dann setzt man der Kultur die in Abschnitt c) hergestellte sterile 17β-Hydroxy-17α-methyl-4-androsten-3-on-Lösung und die in Abschnitt d) hergestellten Zusätze zu. Danach wird weiter 20 Stunden lang fermentiert.

Nach erfolgter Fermentation wird die mit 5 l Äthylenchlorid stabilisierte Kulturbrühe 3 mal mit je 20 l Äthylenchlorid extrahiert und der Äthylenchylorid-Extrakt im Rotationsverdampfer bei max. 50 °C unter Vakuum eingeengt. Anschließend erfolgt eine Reinigung durch Chromatographie an Aluminiumoxyd.

Man erhält 6,2 g 17β-Hydroxy-17α-methyl-1,4-androstadien-3-on vom Schmelzpunkt 163-164,5 °C.

Beispiel 12

a) Ein 2 l-Erlenmeyerkolben mit 1 l steriler Nährlösung enthaltend

0,5 % Cornsteep liquor
0,05 % Glucosemonohydrat
0,1 % Hefeextrakt
— eingestellt auf pH 7,0 —

wird mit einer Abschwemmung einer Trockenkultur von Arthorbacter simplex ATCC 6946 beimpft und bei 30 °C 72 Stunden mit 180 Umdrehungen pro Minute geschüttelt.

b) Ein 50 l Fermenter mit 30 l steriler Nährlösung enthaltend

0,5 % Cornsteep liquor
0,05 % Glucosemonohydrat
0,1 % Hefeextrakt
4 ml Silikon SH
4 ml Pluronic
— eingestellt auf pH 7,0 —

wird mit 1 l Arthrobacter simplex-Anzuchtskultur beimpft und diese Vorkultur bei 30 °C unter Belüftung (2 $m^3$ pro Stunde) und Rühren (220 Umdrehungen pro Minute) 24 Stunden lang incubiert.

c) 6 g 11α,17α-Dihydroxy-16β-methyl-4-pregnen-3,20-dion werden in 150 ml Äthylenglycolmonomethyläther gelöst und anschließend sterilfiltriert.

d) 12 g $CoSO_4 \cdot 7H_2O$ und 12 g $(NH_4)_2SO_4$ werden in 500 ml Wasser gelöst, auf einen pH-Wert von 6,5 eingestellt und sterilisiert.

e) Ein 50 l-Fermenter wird mit 30 l steriler Nährlösung enthaltend

0,5 % Cornsteep liquor
0,05 % Glucosemonohydrat
0,3 % « Proteinhydrolysat »
4 ml Silikon SH
4 ml Pluronic
— eingestellt auf pH 7,0 —

beschickt, mit 3 l Arthrobacter simplex Vorkultur beimpft und unter Belüften (2 $m^3$ pro Stunde) und Rühren (220 Umdrehungen pro Minute) 6 Stunden lang bei 30 °C incubiert. Dann setzt man der Kultur die in Abschnitt c) hergestellte sterile 11α,17α-Dihydroxy-16β-methyl-4-pregnen-3,20-dion-Lösung und die in Abschnitt d) hergestellten Zusätze zu. Danach wird weiter 20 Stunden lang fermentiert.

Nach erfolgter Fermentation wird die mit 5 l Äthylenchlorid stabilisierte Kulturbrühe 3 mal mit je 20 l Äthylenchlorid extrahiert und der Äthylenchlorid-Extrakt im Rotationsverdampfer bei max. 50 °C unter Vakuum eingeengt. Anschließend erfolgt eine Reinigung durch Chromatographie in Aluminiumoxyd.

Man erhält 4,6 g 11α,17α-Dihydroxy-16β-methyl-1,4-pregnadien-3,20-dion vom Schmelzpunkt 203/204-205 °C.

**Patentansprüche**

1. Verfahren zur Herstellung von 3-Oxo-$\Delta^{1,4}$-steroiden dadurch gekennzeichnet, daß man in der 1,2-Position gesättigte 3-Oxo-$\Delta^4$-steroide mit einer lebenden Kultur von Arthrobacter simplex ATCC 6946 in Gegenwart von 0,04 g bis 0,12 g Kobalt(II)-ionen pro Liter Kultur fermentiert.

2. Verfahren zur Herstellung von 3-Oxo-$\Delta^{1,4}$-steroiden der allgemeinen Formel I

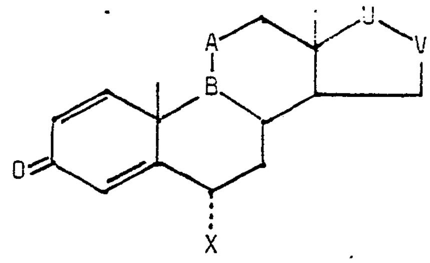

(I)

worin X ein Wasserstoffatom, ein Chloratom, ein Fluoratom der eine Methylgruppe bedeutet,

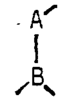

die Gruppierungen

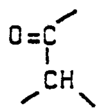 , 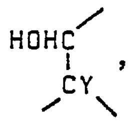 , 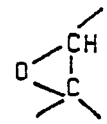 oder 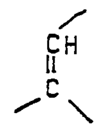

mit Y in der Bedeutung von Wasserstoff, Fluor oder Chlor und

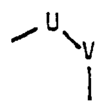

die Gruppierungen

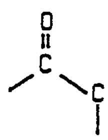 , 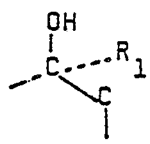 , 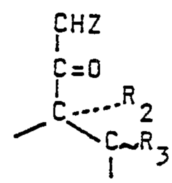 , 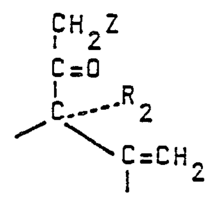

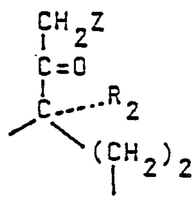 oder 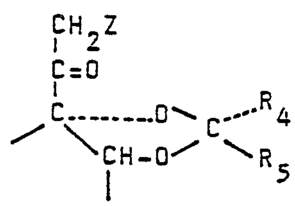

mit

Z in der Bedeutung eines Wasserstoffatoms, einer Hydroxygruppe eines Fluoratoms oder eines Chloratoms,

$R_1$ in der Bedeutung eines Wasserstoffatoms oder eines alicyclischen Kohlenwasserstoffrests mit 1 bis 4 Kohlenstoffatome,

$R_2$ in der Bedeutung eines Wasserstoffatoms, einer Hydroxygruppe, einer gegebenenfalls durch ein Sauerstoffatom oder ein Schwefelatom unterbrochene Alkylgruppe mit 1 bis 6 Kohlenstoffatomen, einer Alkanoyloxygruppe mit 1 bis 6 Kohlenstoffatomen oder einer Benzoyloxygruppe,

$R_3$ in der Bedeutung eines Wasserstoffatoms aus einer Hydroxygruppe oder einer Methylgruppe und

$R_4$ und $R_5$ in der Bedeutung einer Alkylgruppe mit 1 bis 4 Kohlenstoffatomen, darstellen, gemäß Anspruch 1, dadurch gekennzeichnet, daß man ein 3-Oxo-$\Delta^4$-steroid der allgemeinen Formel II

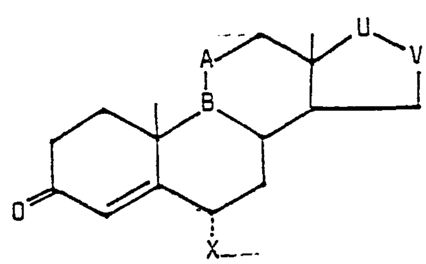

(II)

worin

X, 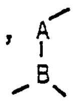 und 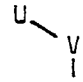

die obengenannte Bedeutung besitzen, oder deren 21-Ester von Alkancarbonsäuren mit 1 bis 6 Kohlenstoffatomen fermentiert.

**Claims**

1. Process for the preparation of 3-oxo-$\Delta^{1,4}$-steroids, characterised in that a 3-oxo-$\Delta^{4}$-steroid, saturated in the 1,2-position, is fermented with a living culture of Arthrobacter simplex ATCC 6946 in the presence of 0.04 to 0.12 g cobalt (II) ion per litre of culture.

2. Process according to claim 1 for the preparation of 3-oxo-$\Delta^{1,4}$-steroids of general formula I

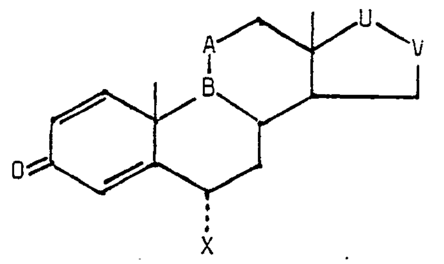

(I)

in which X is hydrogen, chlorine, fluorine or methyl,

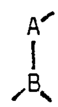

are the groups

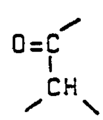 , 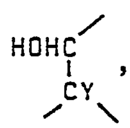 , 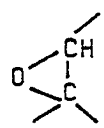 or 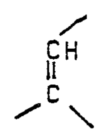

where Y is hydrogen, fluorine or chlorine, and

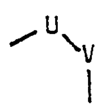

are the groups

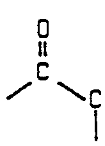 , 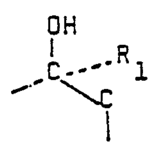 , 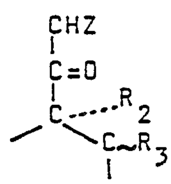 , 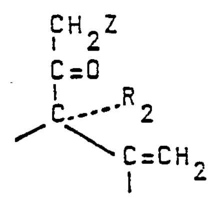

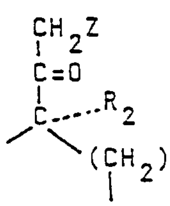 or 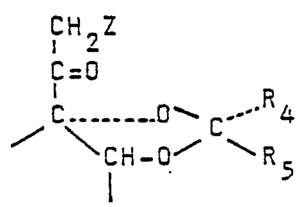

where

Z is hydrogen, hydroxy, fluorine or chlorine,

$R_1$ is hydrogen or an alicyclic hydrocarbon group of 1 to 4 carbon atoms,

$R_2$ is hydrogen, hydroxy, an alkyl group of 1 to 6 carbon atoms, optionally interrupted by oxygen or sulphur, an alkanoyloxy group of 1 to 6 carbon atoms or benzoyloxy,

$R_3$ is hydrogen, hydroxy or methyl, and

$R_4$ and $R_5$ are alkyl of 1 to 4 carbon atoms, characterised in that a 3-oxo-$\Delta^4$-steroid of general formula.

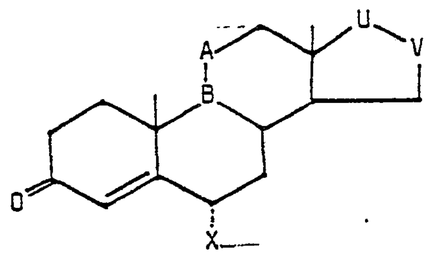

(II)

wherein

X, 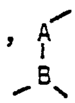 and 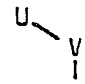

have the meanings given above, or its 21 ester with an alkylcarboxylic acid of 1 to 6 carbon atoms, is fermented.

**Revendications**

1. Procédé pour préparer des stéroïdes de type oxo-3-$\Delta^{1,4}$, caractérisé en ce qu'on fait fermenter les oxo-3-$\Delta^4$-stéroïdes, saturés en position 1,2, avec une culture vivante d'Arthrobacter simplex ATCC 6946 en présence de 0,04 g à 0,12 g d'ions cobalt-(II) par litre de culture.

2. Procédé pour préparer des oxo-3-$\Delta^{1,4}$-stéroïdes de formule générale :

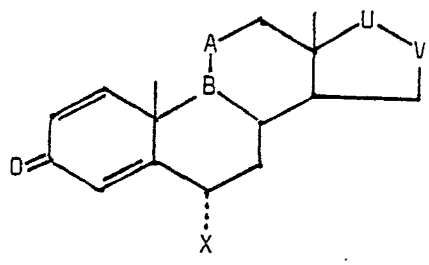

(I)

dans laquelle X représente un atome d'hydrogène, un atome de chlore, un atome de fluor ou un groupe méthyle,

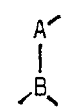

représente le groupement

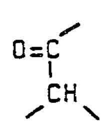 , 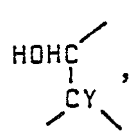 , 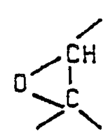 ou 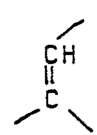

avec Y représentant un atome d'hydrogène, de fluor ou de chlore, et

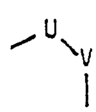

représente les groupements :

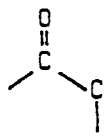 , 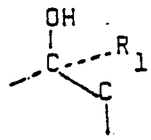 , 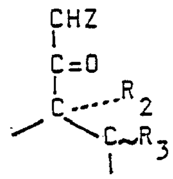 , 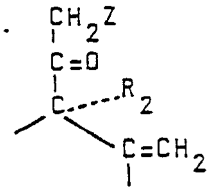

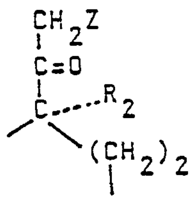 ou 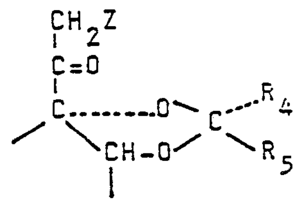

Z a le sens d'un atome d'hydrogène, d'un groupe hydroxyle, d'un atome de fluor ou d'un atome de chlore,

$R_1$ représente un atome d'hydrogène ou un reste d'hydrocarbure alicyclique comportant 1 à 4 atomes de carbone,

$R_2$ a le sens d'un atome d'hydrogène, d'un groupe hydroxyle, d'un groupe alkyle ayant 1 à 6 atomes de carbone et qui est éventuellement interrompu par un atome d'oxygène ou un atome de soufre, d'un groupe alcanoyle comportant 1 à 6 atomes de carbone ou d'un groupe benzoyloxy,

$R_3$ a le sens d'un atome d'hydrogène d'un groupe hydroxyle ou d'un groupe méthyle, et

$R_4$ et $R_5$ représentent un groupe alkyle ayant 1 à 4 atomes de carbone, selon la revendication 1, caractérisé en ce qu'on fait fermenter un oxo-3-$\Delta^4$-stéroïde de formule générale II :

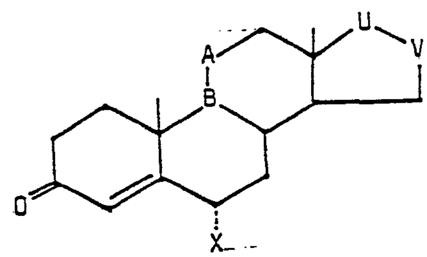

(II)

dans laquelle :

X, 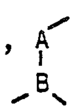 et 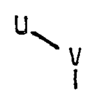

ont les sens précités,
ou leurs esters en 21 d'acides alcanecarboxyliques comportant 1 à 6 atomes de carbone.